# EUROPÄISCHE PATENTSCHRIFT

(11) **EP 1 345 924 B1**
(45) Veröffentlichungstag und Bekanntmachung des Hinweises auf die Patenterteilung: **30.08.2006**
(21) Anmeldenummer: 01991821.8
(22) Anmeldetag: 11.12.2001
(51) Int. Cl.: C07D 401/14, A61K 31/444, A61P 9/10

(54) **DIPHENYLAZETIDINONDERIVATE, VERFAHREN ZU DEREN HERSTELLUNG, DIESE VERBINDUNGEN ENTHALTENDE ARZNEIMITTEL UND DEREN VERWENDUNG**
DIPHENYL AZETIDINONE DERIVATIVES, METHOD FOR THE PRODUCTION THEREOF, MEDICAMENTS CONTAINING THESE COMPOUNDS, AND THEIR USE
DERIVES DE DIPHENYLAZETINONE, PROCEDE PERMETTANT DE LES PRODUIRE, MEDICAMENTS CONTENANT LESDITS COMPOSES ET LEUR UTILISATION

(30) Priorität: 21.12.2000 DE 10064402; 07.11.2001 DE 10154518
(43) Veröffentlichungstag der Anmeldung: 24.09.2003
(73) Patentinhaber: Sanofi-Aventis Deutschland GmbH, 65929 Frankfurt am Main (DE)
(72) Erfinder: GLOMBIK, Heiner, 65719 Hofheim (DE); KRAMER, Werner, 55130 Mainz-Laubenheim (DE); FLOHR, Stefanie, CH-4054 Basel (DE); FRICK, Wendelin, 65510 Hünstetten-Beuerbach (DE); HEUER, Hubert, 55270 Schwabenheim (DE); JAEHNE, Gerhard, 65929 Frankfurt (DE); LINDENSCHMIDT, Andreas, 65812 Bad Soden (DE); SCHAEFER, Hans-Ludwig, 65239 Hochheim (DE)
(86) Internationale Anmeldenummer: PCT/EP2001/014533
(87) Internationale Veröffentlichungsnummer: WO 2002/050060

(56) Entgegenhaltungen:
- EP-A- 0 869 121
- WO-A-00/20393
- WO-A-00/20410
- WO-A-95/35277

## Beschreibung

Die Erfindung betrifft substituierte Diphenylazetidinone, deren physiologisch verträgliche Salze sowie physiologisch funktionelle Derivate.

Es sind bereits Diphenylazetidinone (wie z.B. Ezetimibe) sowie deren Verwendung zur Behandlung von Hyperlipidämie sowie Arteriosklerose und Hypercholesterinämie beschrieben worden [vgl. Drugs of the Future 2000, 25(7):679-685].

Der Erfindung lag die Aufgabe zugrunde, weitere Verbindungen zur Verfügung zu stellen, die eine therapeutisch verwertbare hypolipidämische Wirkung entfalten. lnsbesonders bestand die Aufgabe darin, neue Verbindungen zu finden, die gegenüber den im Stand der Technik beschriebenen Verbindungen sehr gering resorbierbar sind. Unter sehr gering resorbierbar wird eine intestinale Resorption kleiner 10%, bevorzugt kleiner oder gleich 5% verstanden.
Die neuen Verbindungen sollen insbesonders eine geringere Resorption als Ezetimibe auf weisen.
Bei geringerer Resorption zeigen pharmazeutische Wirkstoffe in der Regel deutlich weniger Nebenwirkungen.

Die Erfindung betrifft daher Verbindungen der Formel I, 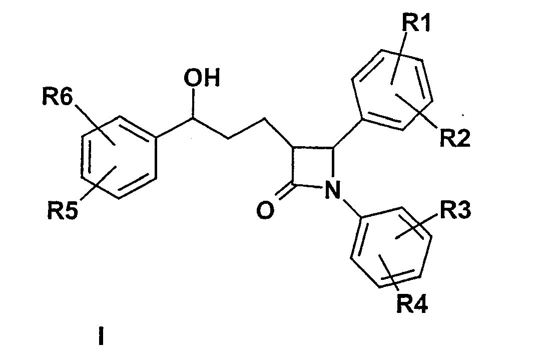 worin bedeuten
- R1, R2, R3, R4, R5, R6: unabhängig voneinander (C₀-C₃₀)-Alkylen-L, wobei ein oder mehrere C-Atome des Alkylenrests, durch eine der Gruppen -O-, -(C=O)-, -CH=CH, -C≡C-, -N((C₁-C₆)Alkyl)- oder -NH- ersetzt sein können,
H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;

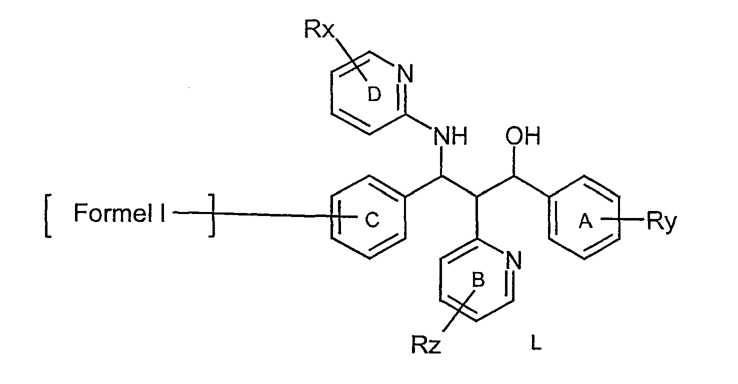

- Rx, Ry, Rz: unabhängig voneinander H, F, Cl, Br, J, CF₃, NO₂, CN, COOH, COO(C₁-C₆)Alkyl, CONH₂, CONH(C₁-C₆)Alkyl, CON[(C₁₋C₆)Alkyl]₂, (C,-C₆)-Alkyl, (C₂-C₆)-Alkenyl, (C₂-C₆)-Alkinyl, 0-(C_{I}-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
wobei immer mindestens einer der Reste R1 bis R6 die Bedeutung (C₀-C₃₀)-Alkylen-L, wobei ein oder mehrere C-Atome des Alkylenrests, durch -O-, - (C=O)-, -CH=CH-, -C≡C-, -N((C₁-C₆)-Alkyl)- oder -NH- ersetzt sein können, besitzen muß,
sowie deren pharmazeutisch verträglichen Salze.

Bevorzugt sind Verbindungen der Formel I, worin mindestens einer der Reste R1 bis R6 die Bedeutung (C₀-C₃₀)-Alkylen-L, wobei ein oder mehrere C-Atome des Alkylenrests, durch -O-, -(C=O)- oder -NH- ersetzt sein können, besitzt.

Besonders bevorzugt sind Verbindungen der Formel I, worin einer der Reste R1 oder R3 die Bedeutung (C₀-C₃₀)-Alkylen-L hat, wobei ein oder mehrere C-Atome des Alkylenrests, durch -O-, -(C=O)- oder -NH- ersetzt sein können.

Ganz besonders bevorzugt sind Verbindungen der Formel I, worin einer der Reste R1 oder R3 die Bedeutung -(CH₂)₀₋₁-NH-(C=O)₀₋₁-(C₃-C₂₅)-Alkylen-(C=O)₀₋₁-NH-L hat, wobei ein oder mehrere C-Atome des Alkylenrests, durch O-Atome ersetzt sein können.

Die Verknüpfung eines der Reste R1 bis R6 mit dem L-Rest erfolgt bevorzugt in Meta-Stellung des Rings C der L-Gruppe.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung sind Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Verbindungen der Formel I und deren pharmazeutisch verträgliche Salze und physiologisch funktionelle Derivate stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen.

Die Verbindung(en) der Formel (I) können auch in Kombination mit weiteren Wirkstoff verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Benzothiepin-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.
Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesonders zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Antidiabetika umfassen Insulin und Insulinderivate, wie z.B. Lantus® oder HMR 1964, GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguadine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlididämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Bay 13-9952, BMS-201038, R-103757, verabreicht.

Bei einer Ausführungsform der Erfindung werden die Verbindungen der Formel I in Kombination mit Gallensäureresorptionsinhibitor, wie z.B. HMR 1453, verabreicht.

Bei einer Ausführungsform der Erfindung werden die Verbindungen der Formel I in Kombination mit einem CETP-Inhibitor, wie z.B. Bay 194789, verabreicht.

Bei einer Ausführungsform der Erfindung werden die Verbindungen der Formel I in Kombination mit einem polymeren Gallensäureadsorber, wie z.B. Cholestyramin, Colesolvam, verabreicht.

Bei einer Ausführungsform der Erfindung werden die Verbindungen der Formel I in Kombination mit einem LDL-Rezeptorinducer, wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen synthetase inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylhamstoff, wie z.B. Tolbutamid, Glibenclamid, Glipizid oder Glicazid, verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinyl-methoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Gliazid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormon, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel 1 in Kombination mit Ballaststoffen, vorzugsweise unlöslichen Ballaststoffen, wie z.B. Caromax® verabreicht. Die Kombination mit Caromax® kann in einer Zubereitung erfolgen, oder durch getrennte Gabe von Verbindungen der Formel I und Caromax® . Caromax® kann dabei auch in Form von Lebensmitteln, wie z.B. in Backwaren oder Müsliriegeln, verabreicht werden. Die Kombination von Verbindungen der Formel I mit Caromak® zeichnet sich neben einer Wirkverbesserung, insbesonders in der LDL-Cholesterinsenkung, gegenüber den Einzelwirkstoffen, auch durch Ihre verbesserte Verträglichkeit aus.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Gegenstand der Erfindung sind weiterhin sowohl Stereoisomerengemische der Formel 1, als auch die reinen Stereoisomere der Formel 1, sowie Diastereomerengemische der Formel I als auch die reinen Diastereomere. Die Trennung der Gemische erfolgt auf chromatographischem Weg. Bevorzugt sind racemische als auch enantiomerenreine Verbindungen der Formel 1 mit folgender Struktur:

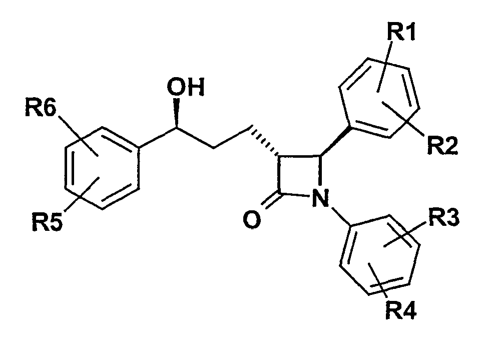

Weiterhin bevorzugt sind Verbindungen der Formel I, worin die L Reste folgende Bedeutung haben:

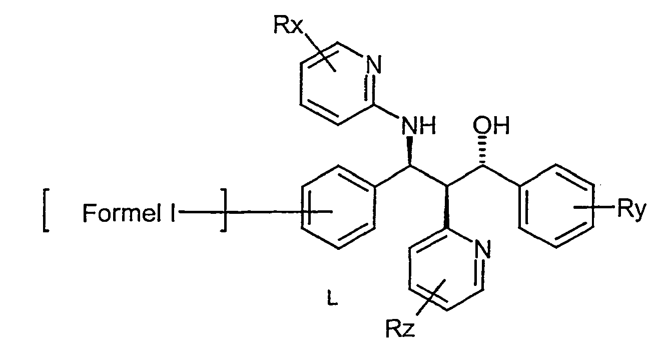

Gegenstand der Erfindung ist weiterhin ein Verfahren zur Herstellung der Verbindungen der allgemeinen Formel 1, dadurch gekennzeichnet, daß man die Verbindungen der Formel I so gewinnt, daß analog dem folgenden Reaktionsschema vorgegangen wird.

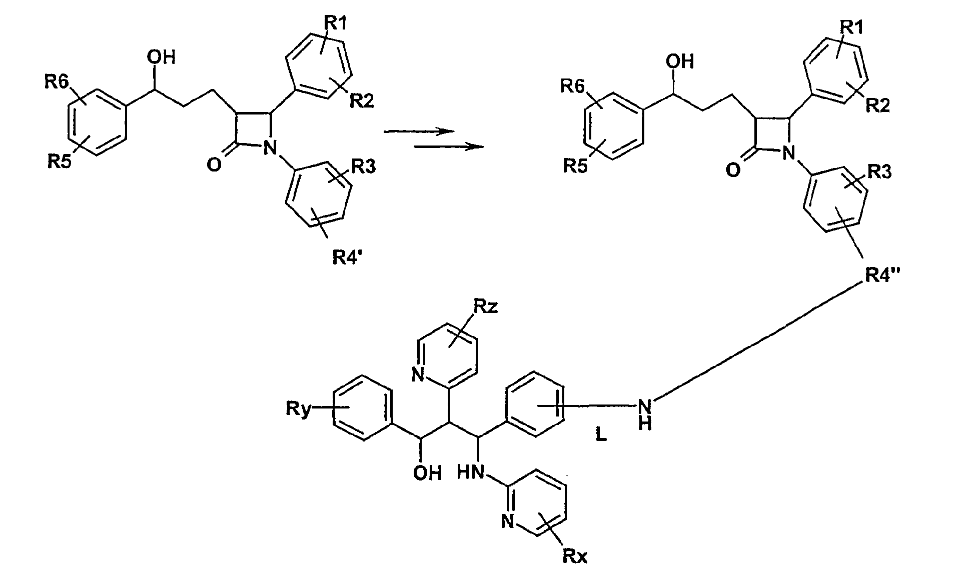

R4" bedeutet (C₀-C₃₀)-Alkylen, wobei ein oder mehrere C-Atome des Alkylenrests, durch -O-, -(C=O)-, -CH=CH-, -C≡C-, -N((C₁-C₆)-Alkyl)- oder-NH- ersetzt sein können.

Alternativ erfolgt die Verknüpfung zur L-Gruppe über Ring A oder Ring C.

Die nachfolgenden Beispiele dienen zur näheren Erläuterung der Erfindung, ohne dieselbe auf in den Beispielen beschriebene Produkte und Ausführungsformen einzuschränken.

### Beispiel IV

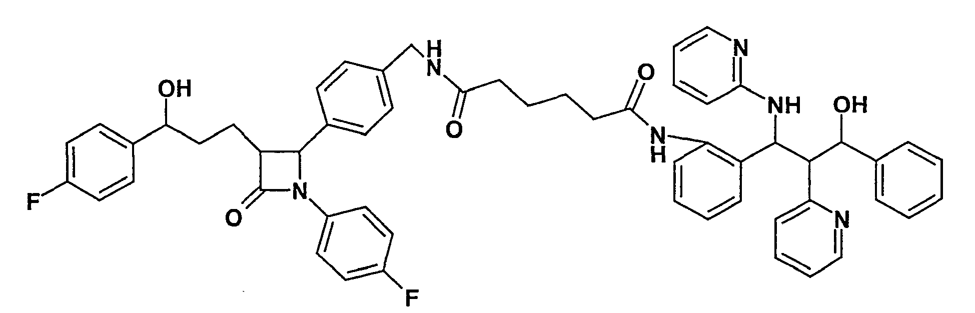

Hexandisäure 4-{1-(4-fluor-phenyl)-3-[3-(4-fluor-phenyl)-3-hydroxy-propyl]-4-oxo-azetidin-2-yl}-benzylamid {2-[3-hydroxy-3-phenyl-2-pyridin-2-yl-1-(pyridin-2-ylamino)-propyl]-phenyl}-amid (14)
a) 5-{2-[3-Hydroxy-3-phenyl-2-pyridin-2-yl-1-(pyridin-2-ylamino)-propyl]-phenylcarbamoyl}- pentansäure (13)
   Herstellung ausgehend von 1.5 g 3-(2-Amino-phenyl)-1-phenyl-2-pyridin-2-yl-3-(pyridin-2-ylamino)-propan-1-ol, 3.4 g Hexandisäure, 1.04 g Dicyclohexylcarbodiimid und 640 mg Benzotriazol-1-ol, analog zu Beispiel II f) Produkt_ mit dem Molekulargewicht 524.6 (C₃₁H₃₂N₄O₄); MS (ESI+): 525 (M+H⁺): (ESI-): 523 (M-H⁺)
b) Hexandisäure 4-{1-(4-fluor-phenyl)-3-[3-(4-fluor-phenyl)-3-hydroxy-propyl]-4-oxo-azetidin-2-yl}-benzylamid {2-[3-hydroxy-3-phenyl-2-pyridin-2-yl-1-(pyridin-2-ylamino)-propyl]-phenyl}-amid (14)
   Herstellung ausgehend von 300 mg 4-(4-Aminomethyl-phenyl)-1-(4-fluor-phenyl)-3-[3-(4-fluor-phenyl)-3-hydroxy-propyl]- azetidin-2-on, 372 mg 5-{2-[3-Hydroxy-3-phenyl-2-pyridin-2-yl-1-(pyridin-2-ylamino)-propyl]-phenylcarbamoyl}- pentansäure, 155 mg Dicyclohexyl-carbodümid und 120 mg Benzotriazol-1-ol in 25 ml Tetrahydrufuran analog Beispiel III, Chromatographie: SiO₂, Dichlormethan/Methanol = 10:1 ; Produkt mit dem Molekulargewicht 929.1 (Cti6H₅₄F₂N60₅), MS (ESI+): 929 (M+H⁺).

### Beispiel XIII

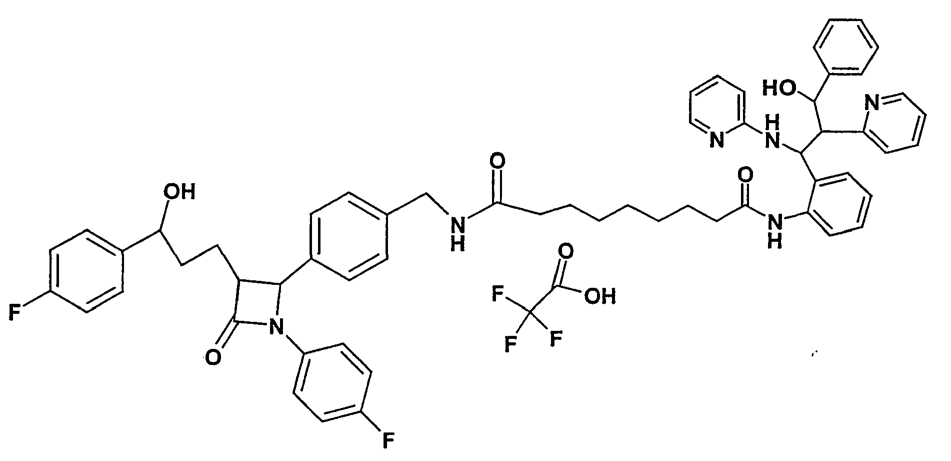

(1-{2-[8-(4-{1-(4-Fluor-phenyl)-3-[3-(4-fluor-phenyl)-3-hydroxy-propyl]-4-oxo-azetidin-2-yl}-benzylcarbamoyl)-octanoylamino]-phenyl}-3-hydroxy-3-phenyl-2-pyridin-2-yl-propyl)- pyridin-2-yl-ammonium; trifluor-acetat (27) 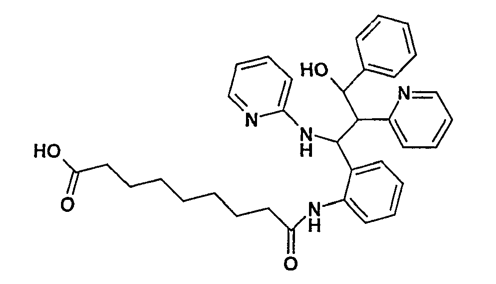
a) 8-{2-[3-Hydroxy-3-phenyl-2-pyridin-2-yl-1-(pyridin-2-ylamino)-propyl]-phenylcarbamoyl}- octansäure (26)
   C₃₄H₃sN₄0₄ (566.72) MS (ESI) 567 (M + H) 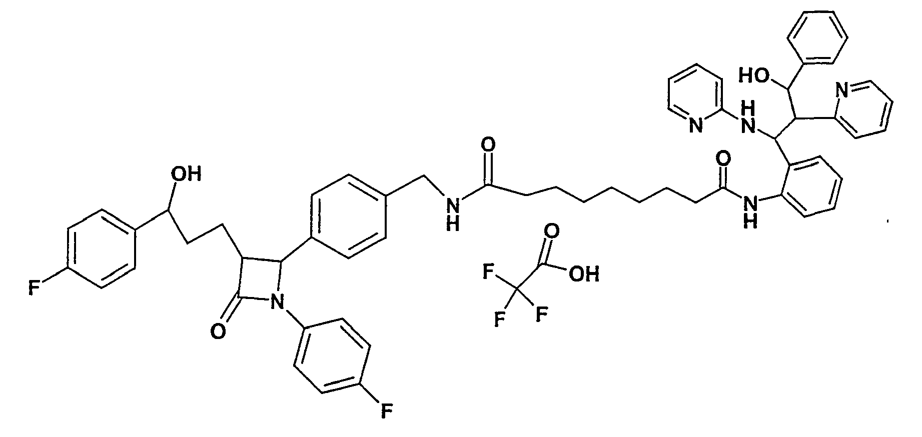
b) (1-{2-[8-(4-{1-(4-Fluor-phenyl)-3-[3-(4-fluor-phenyl)-3-hydroxy-propyl]-4-oxo-azetidin- 2-yl}-benzylcarbamoyl)-octanoylamino]-phenyl}-3-hydroxy-3-phenyl-2-pyridin-2-yl-propyl)- pyridin-2-yl-ammonium; trifluor-acetat (27)
   C₆₁H₆₁F₅N₆O₇ (1085,19) MS (ESI) 971 (M + H)

### Beispiel XIV

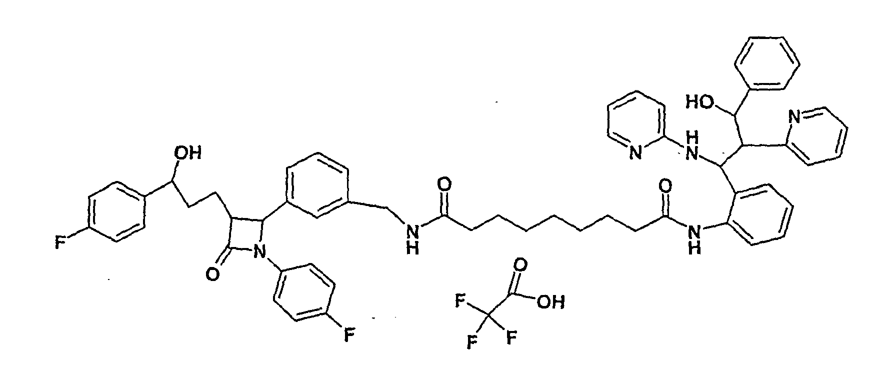

{1-{2-[8-(3-{1-(4-Fluor-phenyl)-3-[3-(4-fluor-phenyl)-3-hydroxy-propyl]-4-oxo-azetidin-2-yl}-benzylcarbamoyl)-octanoylamino]-phenyl)-3-hydroxy-3-phenyl-2-pyridin-2-yl-propyl)- pyridin-2-yl-ammonium; trifluor-acetat (28)

C₆₁H₆₁F₅N₆O₇ (1085.19) MS (ESI) 971 (M + H)

### Beispiel XV

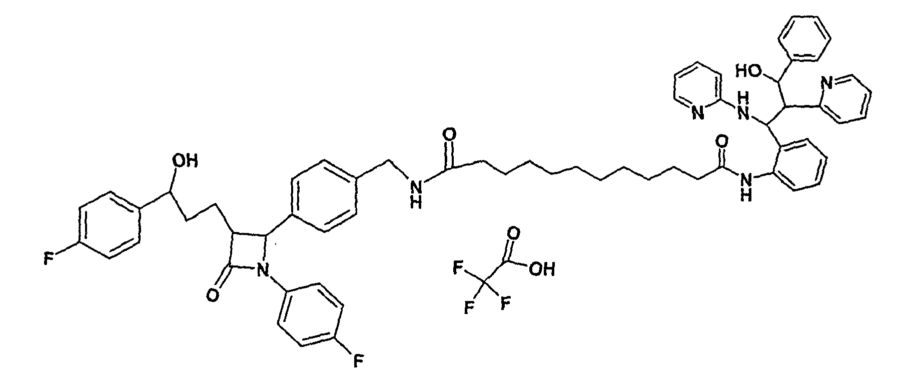

(1-{2-[11-(4-{1-(4-Fluor-phenyl)-3-(3-(4-fluor-phenyl)-3-hydroxy-propyl]-4-oxo-azetidin- 2-yl}-benzylcarbamoyl-undecanoylamino]-phenyl}-3-hydroxy-3-phenyl-2-pyridin-2-yl-propyl)- pyridin-2-yl-ammonium; trifluor-acetat (30) 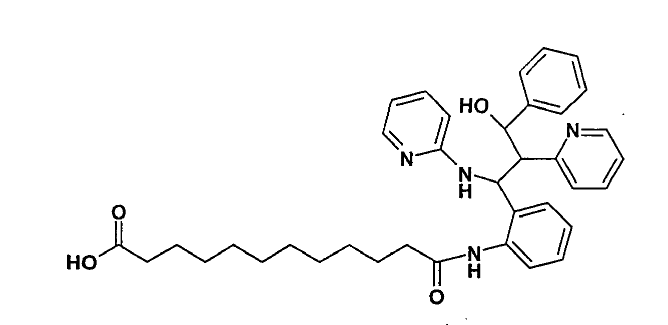
a) 11-{2-[3-Hydroxy-3-phenyl-2-pyridin-2-yl-1-(pyridin-2-ylamino)-propyl]-phenylcarbamoyl}- undecansäure (29)
   C₃₇H₄₄N₄O₄ (608.82) MS (ESI) 609 (M + H) 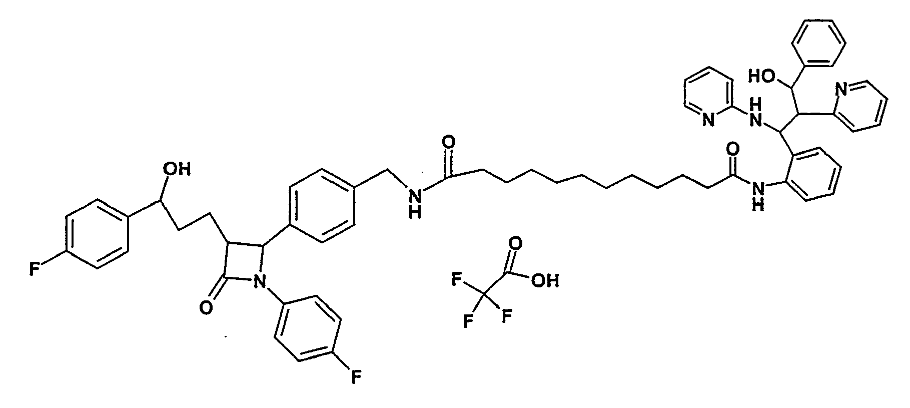
b) (1-{2-[11-(4-{1-(4-Fluor-phenyl)-3-[3-(4-fluor-phenyl)-3-hydroxy-propyl]-4-oxo-azetidin- 2-yl}-benzylcarbamoyl)-undecanoylamino]-phenyl}-3-hydroxy-3-phenyl-2-pyridin-2-yl-propyl)- pyridin-2-yl-ammonium; trifluor-acetat (30)
   C₆₄H₆₇F₅N₆O₇ (1127,28) MS (ESI) 1013 (M + H)

### Beispiel XVI

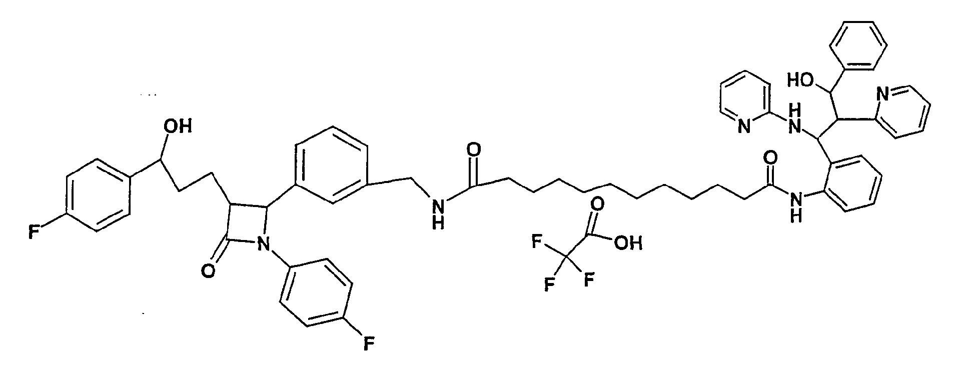

(1-{2-[11-(3-{1-(4-Fluor-phenyl)-3-[3-(4-fluor-phenyl)-3-hydroxy-propyl]-4-oxo-azetidin- 2-yl}-benzylcarbamoyl)-undecanoylamino]-phenyl}-3-hydroxy-3-phenyl-2-pyridin-2-yl-propyl)- pyridin-2-yl-ammonium; trifluor-acetat (31)

C₆₄H₆₇F₅N₆O₇ (1127.28) MS (ESI) 1013 (M + H)

### Beispiel XVII

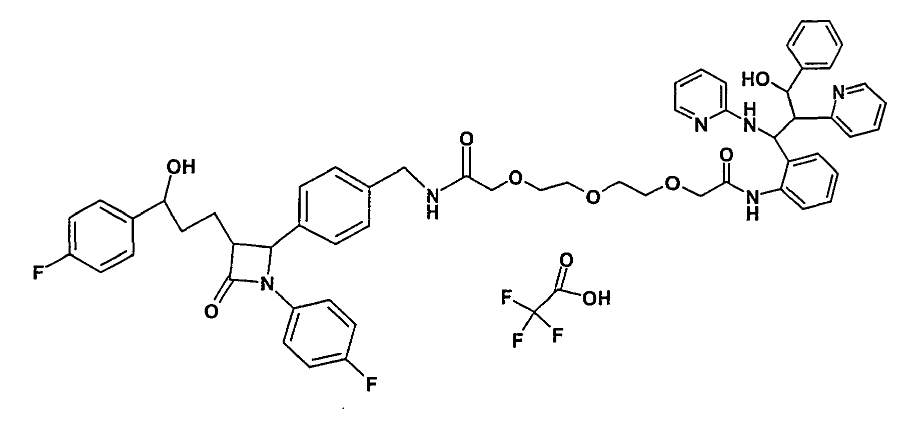

(1-{2-[2-(2-{2-[(4-{1-(4-Fluor-phenyl)-3-[3-(4-fluor-phenyl)-3-hydroxy-propyl]-4-oxo-azetidin-2-yl}-benzylcarbamoyl)-methoxy]-ethoxy}-ethoxy)-acetylamino]-phenyl}-3-hydroxy-3- phenyl-2-pyridin-2-yl-propyl)-pyridin-2-yl-ammonium; trifluor-acetat (33) 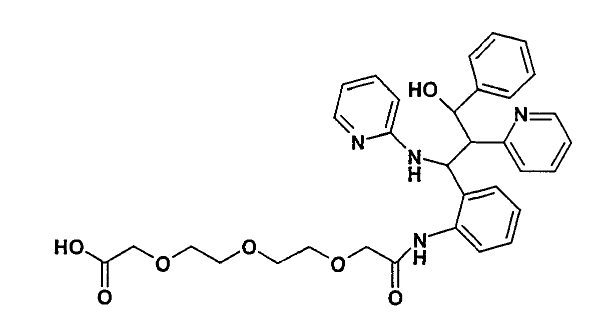
a) {2-[2-({2-[3-Hydroxy-3-phenyl-2-pyridin-2-yl-1-(pyridin-2-ylamino)-propyl]-phenylcarbamoyl}-methoxy)-ethoxy]-ethoxy}-essigsäure (32)
   C₃₃H₃₆N₄O₇ (600.68) MS (ESI) 591 (M + H) 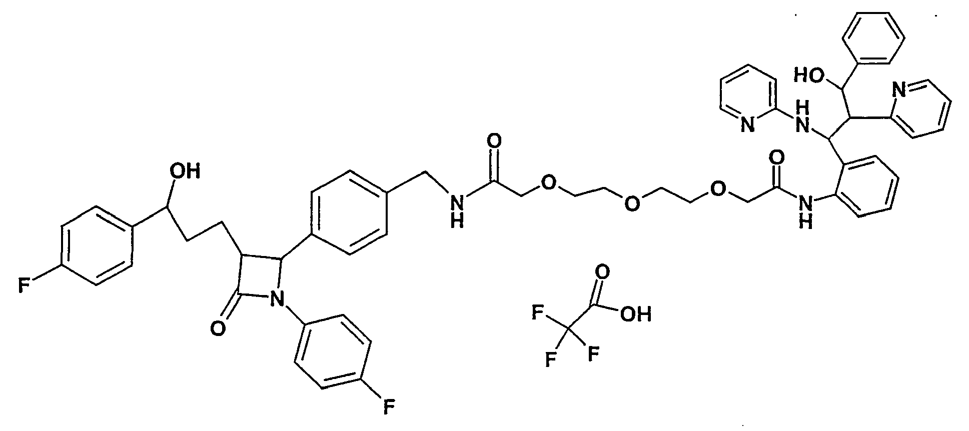
b) (1-{2-[2-(2-{2-[(4-{1-(4-Fluor-phenyl)-3-[3-(4-fluor-phenyl)-3-hydroxy-propyl]-4-oxo-azetidin-2-yl}-benzylcarbamoyl)-methoxy]-ethoxy}-ethoxy)-acetylamino]-phenyl}-3-hydroxy-3- phenyl-2-pyridin-2-yl-propyl)-pyridin-2-yl-ammonium; trifluor-acetat (33)
   C₆₀H₅₉F₅N₆O₁₀ (1119.17) MS (ESI) 1005 (M + H)

### Beispiel XVIII

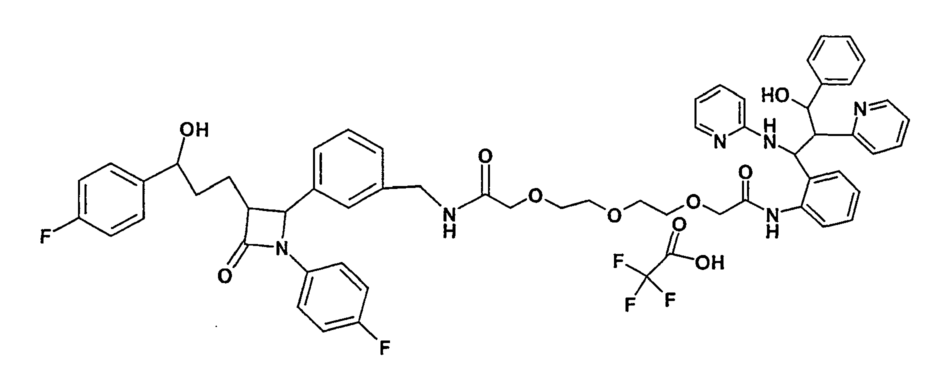

(1-{2-[2-(2-{2-[(3-{1-(4-Fluor-phenyl)-3-[3-(4-fluor-phenyl)-3-hydroxy-propyl]-4-oxo-azetidin-2-yl}-benzylcarbamoyl)-methoxy]-ethoxy}-ethoxy)-acetylamino]-phenyl}-3-hydroxy-3- phenyl-2-pyridin-2-yl-propyl)-pyridin-2-yl-ammonium; trifluor-acetat (34)

C₆₀H₅₉F₅N₆O₁₀ (1119.17) MS (ESI) 1005 (M + H)

### Beispiel XIX

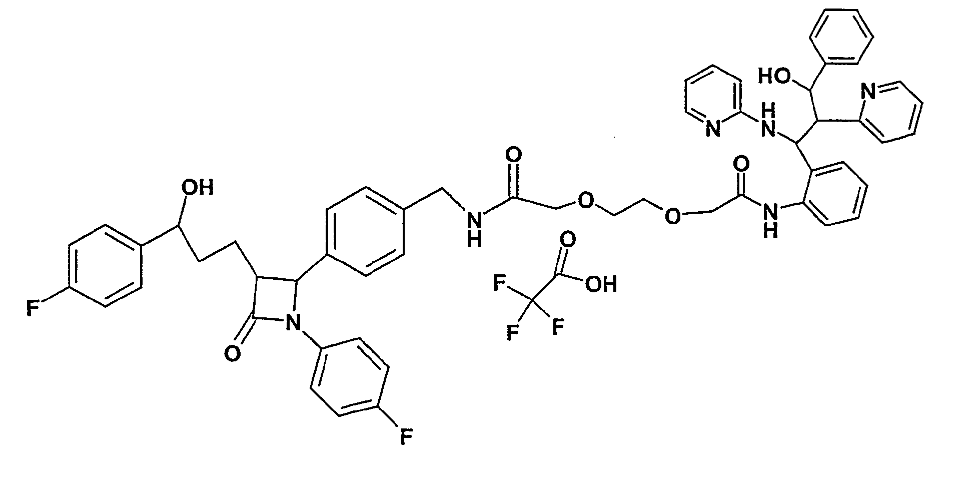

{1-[2-(2-{2-[(4-{1-(4-Fluor-phenyl)-3-[3-(4-fluor-phenyl)-3-hydroxy-propyl]-4-oxo-azetidin-2-yl}-benzylcarbamoyl)-methoxy]-ethoxy}-acetylamino)-phenyl]-3-hydroxy-3-phenyl- 2-pyridin-2-yl-propyl}-pyridin-2-yl-ammonium; trifluor-acetat 36

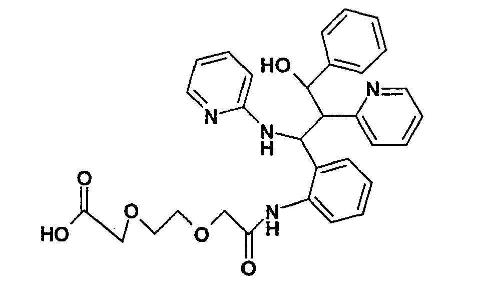

C₃₁H₃₂N₄O₆ (556.62) MS (ESI) 557 (M + H)
a) 2-({2-[3-Hydroxy-3-phenyl-2-pyridin-2-yl-1-(pyridin-2-ylamino)-propyl]-phenylcarbamoyl}- methoxy)-ethoxy]-essigsäure (35) 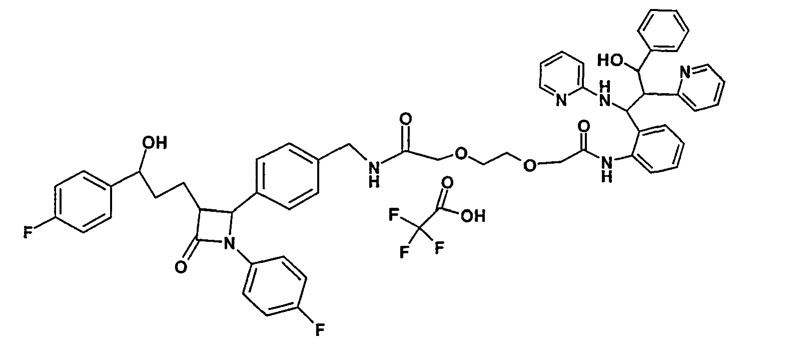
b) {1-[2-(2-{2-[(4-{1-(4-Fluor-phenyl)-3-[3-(4-fluor-phenyl)-3-hydroxy-propyl]-4-oxo-azetidin-2-yl}-benzylcarbamoyl)-methoxy]-ethoxy]-acetylamino)-phenyl]-3-hydroxy-3-phenyl- 2-pyridin-2-yl-propyl}-pyridin-2-yl-ammonium; trifluor-acetat (36)
   C₅₈H₅₅F₅N₆O₉ (1075.11) MS (ESI) 961 (M + H)

### Beispiel XX

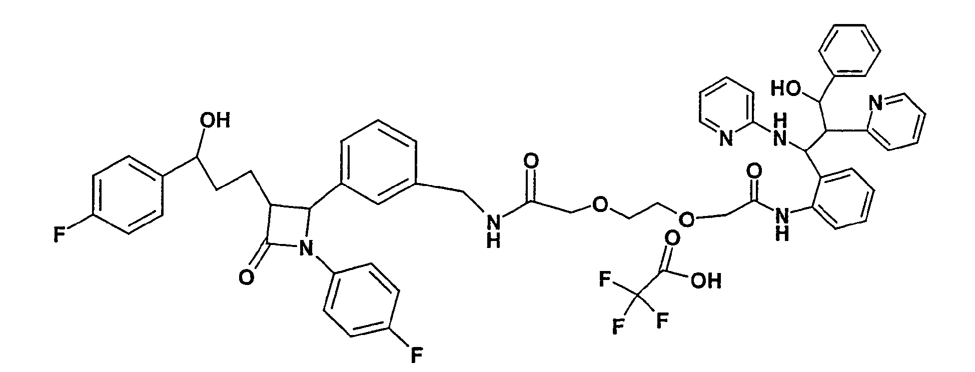

{1-[2-(2-{2-[(3-{1-(4-Fluor-phenyl)-3-[3-(4-fl uor-phenyl)-3-hydroxy-propyl]-4-oxo-azetidin-2-yi}-benzylcarbamoyl)-methoxy]-ethoxy}-acetyfamino)-phenyl]-3-hydroxy-3-phenyl- 2-pyridin-2-yl-propyll-pyridin-2-yl-ammonium; trifluor-acetat (37)

C₅₈H₅₅F₅N₆0₉ (1075.11) MS (ESI) 961 (M + H)

Die erfindungsgemäßen Verbindungen der Formel wurden mit der nachfolgend beschriebenen Methode auf ihre Wirkung geprüft:

### Beeinflussung der Cholesterolabsorption + ³H-Taurocholsäureausscheidung anhand der fäkalen Ausscheidung an der Maus, Ratte oder Hamster

NMRI- Mäuse, Wistar-Ratten, oder Golden Syrian Hamster (in Gruppen von n=4-6) werden unter Standarddiät (Altromin, Lage (Lippe)) in Stoffwechselkäfigen gehalten. Am Nachmittag vor Gabe der radioaktiven Tracer (¹⁴C-Cholesterol) werden die Tiere nüchtern gesetzt und auf Gitterroste adaptiert.

Zusätzlich werden die Tiere werden 24 Stunden vor der peroralen Applikation der Testmahlzeit C⁴C-Cholesterol in Intralipid® 20, Pharmacia-Upjohn) mit ³H-TCA (Taurocholic acid) s.c. gelabelt (z.b. 1 µCi/Maus bis 5 µCi/Ratte)

Cholesterolabsorptionstest: 0,25 ml/Maus Intralipid ® 20 (Pharmacia- Upjohn) ((Spikung mit 0,25 uCi ¹⁴ C-Cholesterol in 0,1 mg Cholesterol) werden peroral mit der Schlundsonde verabreicht.

Testsubstanzen werden getrennt in 0,5 %/ (Methylcellulose (Sigma)/5% Solutol (BASF, Ludwigshafen) oder geeignetem Vehikel angesetzt.
Das Applikationsvolumen der Testsubstanz beträgt 0,5 ml/Maus. Die Testsubstanz wird unmittelbar vor der Testmahlzeit (Intralipid mit ¹⁴C-Cholesterol-label) (Cholesterolabsorptionstest) appliziert.

Der Kot wird über 24 h gesammelt: die fäkale Elimination von ¹⁴C-Cholesterol und ³H Taurocholsäure (TCA) nach 24 Std. wird bestimmt.

Die Lebern werden entnommen, homogenisiert und Aliquots im Oximaten (Model 307, Packard) verbrannt zur Bestimmung der aufgenommenn/resorbierten Menge an ¹⁴C- Cholesterol.

### Auswertung:

### Kotproben:

Gesamtgewicht bestimmen, mit Wasser auf definiertes Volumen auffüllen, dann homogenisieren, Aliquot eintrockenen und im Oximat (Model 307, Packard zur Verbrennung von radioaktiv gelabelten Proben) verbrennen: Die Menge von radioaktiv ³H- H2O und ¹⁴C- CO2 wird hochgerechnet auf die ausgeschiedene Menge an ³H-Taurocholsäure bzw. ¹⁴C-Cholesterol (Dual-Isotopen-Technik ). Die ED₂₀₀-Werte werden als Dosis aus einer Dosiswirkungskurve interpoliert als diejenige Dosen, die die Auscheidung an TCA bzw. Cholesterol verdoppeln, bezogen auf eine zeitgleich behandelte Kontrollgruppe.

### Leberproben:

Die aufgenommene Menge von ¹⁴C-Cholesterols in die Leber wird bezogen auf die applizierte Dosis. Die ED₅₀ Werte werden interpoliert aus einer Dosiswirkungskurve als diejenige Dosis, die die Aufnahme von ¹⁴C- Cholesterol in die Leber halbiert (50%), bezogen auf eine Kontrollgruppe

Die folgenden ED₅₀ -Werte belegen die Aktivität der erfindungsgemäßen Verbindungen der Formel I

| **Beispiel Nr.** | **ED**_{**50**} **(Leber) [mg/Maus]** |
|---|---|
| **XVI** | **0.03** |
| **XVIII** | **0.3** |
| **XIX** | **0.1** |

Aus der Tabelle ist abzulesen, daß die Verbindungen der Formel I eine sehr gute Cholesterin senkende Wirkung besitzen.

### Resorbierbarkeit:

Die Resorbierbarkeit der Verbindungen der Formel wurde Caco-Zellmodell geprüft (A.R. Hilgers et al., Caco-2 cell monolayers as a model for drug transport across the intestinal mucosa, Pharm. Res. 1990 , 7, 902).

Aus den Meßdaten ist abzulesen, daß die erfindungsgemäßen Verbindungen der Formel I gegenüber den im Stand der Technik beschriebenen Verbindungen (Referenzstruktur) eine deutlich geringere Resorption aufweisen:

| | Referenzstruktur | Beispiel |
|---|---|---|
| Apparenter Partitionskoeffizient Pₐₚₚ [cm/s] (entsprechend Lit. Hilgers) | 4.88 x 10⁻⁰⁶ | |
| Abgeschätzte Human-Resorption | 100% | |

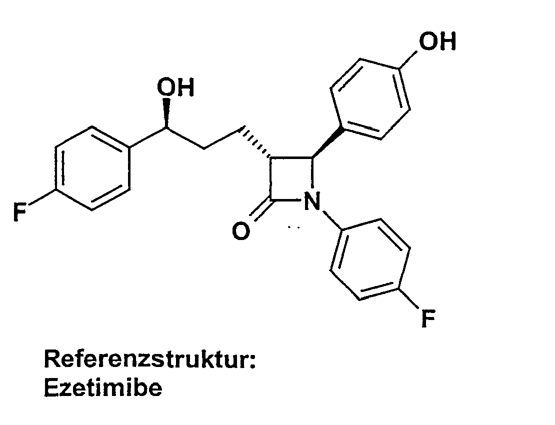

## Patentansprüche

1. Verbindungen der Formel 1, 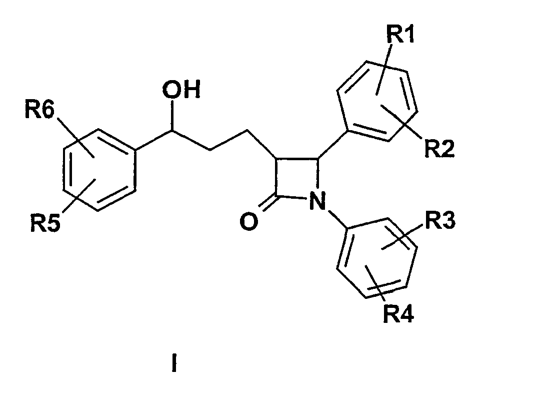 worin bedeuten
R1, R2, R3, R4, R5, R6 unabhängig voneinander (C₀-C₃₀)-Alkylen-L, wobei ein oder mehrere C-Atome des Alkylenrests, durch -O-, -(C=O)-, -CH=CH-, -C≡C-, -N((C₁-C₆)-Alkyl)- oder -NH- ersetzt sein können, H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, CI, Br, J, OH, CF₃, N0₂, CN, OCF₃, O-(c₁-C₆)-Alkyl, (C₁-C₆)-Alkyl, NH₂, NH(C₁-C₆)-Alkyl, N((C₁-C₆)-Alkyl)₂, S0₂-CH₃, COOH, COO-(C₁-C₆)-Alkyl, CONH₂;
L 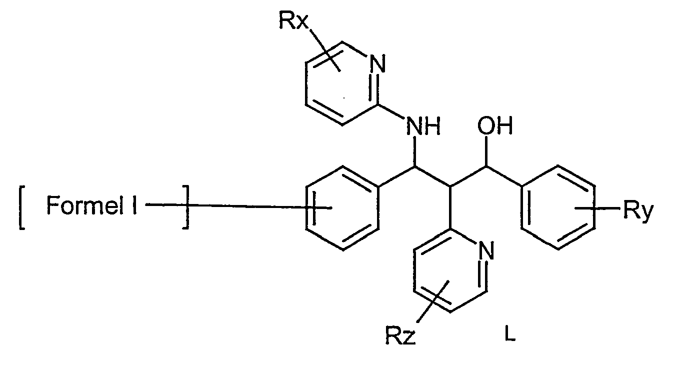
Rx, Ry, Rz unabhängig voneinander H, F, Cl, Br, J, CF₃, N0₂, CN, COOH, COO(C₁-C₆)Alkyl, CONH₂, CONH(C₁-C₆)Alkyl, CON[(C₁₋C₆)Alkyl]₂, (C₁-C₆)-Alkyl, (C₂-C₆rAlkenyl, (C₂-C₆-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
wobei immer mindestens einer der Reste R1 bis R6 die Bedeutung (C₀-C₃₀)-Alkylen-L, wobei ein oder mehrere C-Atome des Alkylenrests, durch -O-, - (C=O)-, -CH=CH-, -C≡C-, -N(C₁-C₆)-Alkyl)- oder -NH- ersetzt sein können, besitzen muß,
sowie deren pharmazeutisch verträglichen Salze.

2. Verbindungen der Formel I, gemäß Anspruch 1, **dadurch gekennzeichnet, daß** darin bedeuten
R1, R2, R3, R4, R5, R6 unabhängig voneinander (C₀-C₃₀)-Alkylen-L, wobei ein oder mehrere C-Atome des Alkylenrests durch -O-, -(C=O)- oder -NH- ersetzt sein können, H, F, Cl, Br, J, CF₃, NO₂, CN, COOH, COO(C₁-C₆)Alkyl, CONH₂, CONH(C₁-C₆)Alkyl, CON[(C₁-C₆)Alk_{Y}11₂. (C₁-C₆)-Alkyl, (C₂-C₆)-Alkenyl, (C₂-C₆)-Alkinyl, O-(C₁-C₆)Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
L 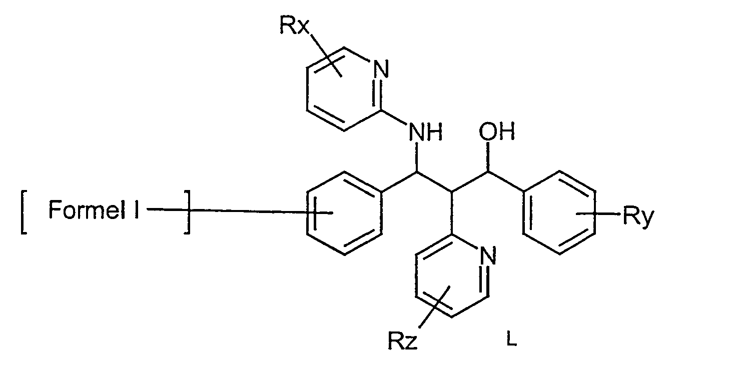
Rx, Ry, Rz unabhängig voneinander H, F, Cl, Br, J, CF₃, NO₂, CN, COOH, COO(C₁-C₆)Alky), CONH₂, CONH(C₁-C₆)Alkyl, CON[(C₁₋C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
wobei immer mindestens einer der Reste R1 bis R6 die Bedeutung (C₀-C₃₀)-Alkylen-L, wobei ein oder mehrere C-Atome des Alkylenrests durch -O-, - (C=O)- oder -NH- ersetzt sein können, besitzt,
sowie deren pharmazeutisch verträglichen Salze.

3. Verbindungen der Formel I, gemäß Anspruch 1 oder 2, **dadurch gekennzeichnet, daß** darin bedeuten
R1, R2, R3, R4, R5, R6 unabhängig voneinander (C₀-C₃₀)-Alkylen-L, wobei ein oder mehrere C-Atome des Alkylenrests durch -O-, -(C=O)- oder -NH- ersetzt sein können,
H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C6)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, N0₂, CN, OCF₃, O-(C₁-C₆Alkyl, (C₁-C₆)Alkyl, NH₂ substituiert sein kann;
NH₂, NH-(C₁-C₆)-Alkyl, N((C,-C₆)-Alkyl)₂, NH(C₁-C₇)-Acyl, Phenyl, O-(CH₂)II-Phenyl, wobei n = 0 - 6 sein kann, wobei der Phenylring ein bis 3-fach substituiert sein kann mit F, CI, Br, J, OH, CF₃, NO₂, CN, OCF₃, O-(C₁-C₆)-Alkyl, (C₁-C₆)Alkyl, NH₂, NH(C₁-C₆)-Alkyl, N((C₁-C₆)-Alkyl)₂, SO₂-CH₃, COOH, COO-(C₁-C₆)-Alkyl, CONH₂;
L 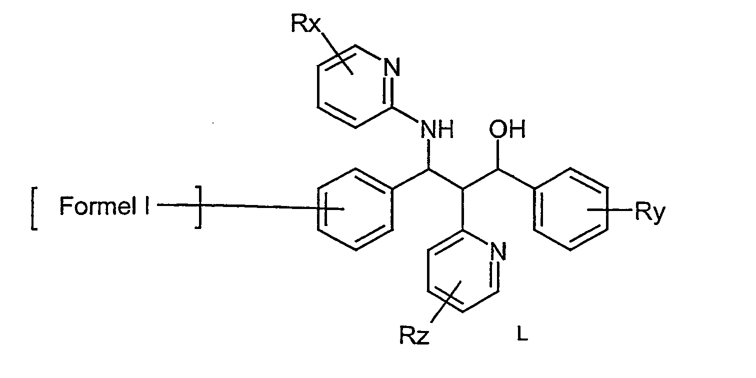
Rx, Ry, Rz unabhängig voneinander H, F, Cl, Br, J, CF₃, NO₂, CN, COOH, COO(C₁-C₆)Alkyl, CONH₂, CONH(C₁-C₆)Alkyl, CON[(C₁₋C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
wobei einer der Reste R1 oder R3 die Bedeutung (C₀-C₃₀)-Alkylen-L, wobei ein oder mehrere C-Atome des Alkylenrests durch -O-, -(C=O)- oder -NH- ersetzt sein können, besitzt,
sowie deren pharmazeutisch verträglichen Salze.

4. Verbindungen der Formel 1 gemäß einem oder mehreren der Ansprüche 1 bis 3, **dadurch gekennzeichnet, daß** darin bedeuten
R1, R2, R3, R4, R5, R6 unabhängig voneinander -(CH₂)₀₋₁-NH-(C=O)₀₋₁-(C₃-C₂₅)-Alkylen-(C=O)₀₋₁-NH-L ,wobei ein oder mehrere C-Atome des Alkylenrests durch O-Atome ersetzt sein können,
H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)Alkyl, CONH₂;
L 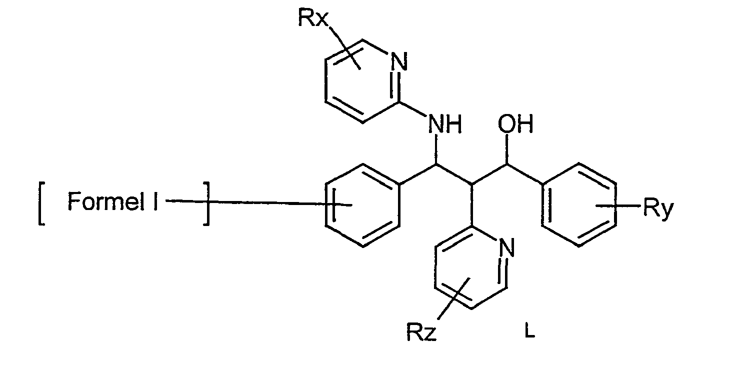
Rx, Ry, Rz unabhängig voneinander H, F, Cl, Br, J, CF₃, N0₂, CN, COOH, Coo(C₁-C₆)Alkyl, CONH₂, CONH(C_{I}-C₆)Alkyl, CON[(C₁₋C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl , S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)2, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
worin einer der Reste R1 oder R3 die Bedeutung -(CH₂)₀₋₁-NH-(C=O)₀₋₁-(C₃-C₂₅)-Alkylen-(C=O)₀₋₁-NH-L ,wobei ein oder mehrere C-Atome des Alkylenrests durch O-Atome ersetzt sein können, besitzt,
sowie deren pharmazeutisch verträglichen Salze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und mindestens einen weiteren Wirkstoff.

7. Arzneimittel, gemäß Anspruch 6, **dadurch gekennzeichnet, daß** es als weiteren Wirkstoff eine oder mehrere Verbindungen, die den Lipidstoffwechsel normalisieren, enthält.

8. Arzneimittel, gemäß Anspruch 6 oder 7, **dadurch gekennzeichnet, daß** es als weiteren Wirkstoff eine oder mehrere
Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

9. Verbindungen gemäß einem oder mehreren der Ansprüche 1 bis 4 zur Anwendung als Medikament zur Behandlung von Lipidstoffwechselstörungen:

10. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindungen gemäß einem oder mehreren der Ansprüche 1 bis 4 zur Herstellung eines Medikaments zur Behandlung von Hyperlipidämie.

12. Verwendung der Verbindungen gemäß einem oder mehreren der Ansprüche 1 bis 4 zur Herstellung eines Medikaments zur Senkung des Serumcholesterinspiegels.

13. Verwendung der Verbindungen gemäß einem oder mehreren der Ansprüche 1 bis 4 zur Herstellung eines Medikament zur Behandlung arteriosklerotischer Erscheinungen.

14. Verwendung der Verbindungen gemäß einem oder mehreren der Ansprüche 1 bis 4 zur Herstellung eines Medikaments zur Behandlung von Insulin Resistenz.

## Claims

1. A compound of the formula I, 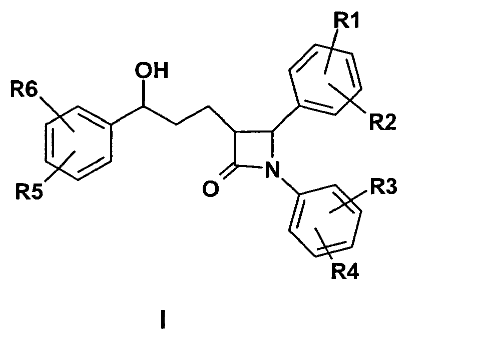 in which
R1, R2, R3, R4, R5, R6 independently of one another are (C₀-C₃₀)-alkylene-L, where one or more carbon atoms of the alkylene radical may be replaced by -O-, -(C=O)-, -CH=CH-, -C≡C-, -N((C₁-C₆)-alkyl)- or -NH-;
H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one or more hydrogens in the alkylene radicals may be replaced by fluorine;
SO₂-NH₂, SO₂NH(C₁-C₆)-alkyl, SO₂N[(C₁-C₆)-alkyl]₂ , S-(C₁-C₆)-alkyl, S-(CH₂)n-phenyl, SO-(C₁-C₆)-alkyl, SO-(CH₂)ₙ-phenyl, SO₂₋(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂; 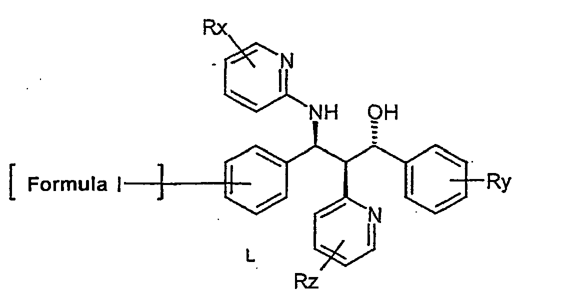
Rx, Ry, Rz independently of one another are H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where in the alkyl radicals one, more or all hydrogen(s) may be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0-6 and the phenyl radical may be substituted up to two times by F, Cl, Br, OH, CF₃, NO₂, CN, OCF₃, O-(C₁-C₆)-alkyl, (C₁-C₆)-alkyl, NH₂; NH₂, NH-(C₁-C₆)-alkyl, N((C₁-C₆)-alkyl)₂, NH(C₁-C₇)-acyl, phenyl, O-(CH₂)ₙ-phenyl, where n = 0-6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
where in each case at least one of the radicals R1 to R6 must have the meaning
(Cₒ-C₃₀)-alkylene-L, where one or more carbon atoms of the alkylene radical may be replaced by -O-, -(C=O)-, -CH=CH-, -C=C-, -N((C₁-C₆)-alkyl)- or-NH-, and its pharmaceutically acceptable salts.

2. A compound of the formula I, as claimed in claim 1, wherein
R1, R2, R3, R4, R5, R6 independently of one another are (C₀-C₃₀)-alkylene-L, where one or more carbon atoms of the alkylene radical may be replaced by -O-, -(C=O)- or -NH-;
H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one or more hydrogens in the alkylene radicals may be replaced by fluorine; SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂₋(C₁-C₆)-alkyl , SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
L 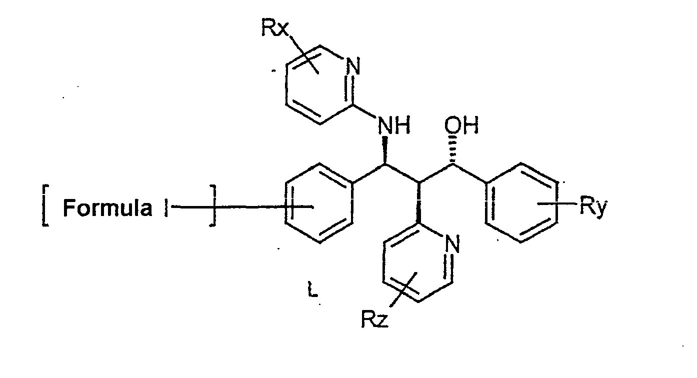
Rx, Ry, Rz independently of one another are H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl , where in the alkyl radicals one, more or all hydrogen(s) may be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0-6 and the phenyl radical may be substituted up to two times by F, Cl, Br, OH, CF₃, NO₂, CN, OCF₃, O-(C₁-C₆)-alkyl, (C₁-C₆)-alkyl, NH₂; NH₂, NH-(C₁-C₆)-alkyl, N((C₁-C₆)-alkyl)₂, NH(C₁-C₇)-acyl, phenyl, O-(CH₂)ₙ-phenyl, where n = 0-6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
where in each case at least one of the radicals R1 to R6 must have the meaning
(C₀-C₃₀)-alkylene-L, where one or more carbon atoms of the alkylene radical may be replaced by -O-, -(C=O)- or -NH-, and its pharmaceutically acceptable salts.

3. A compound of the formula I, as claimed in claim 1 or 2, wherein
R1, R2, R3, R4, R5, R6 independently of one another are (C₀-C₃₀)-alkylene-L, where one or more carbon atoms of the alkylene radical may be replaced by -O-, -(C=O)- or -NH-;
H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one or more hydrogens in the alkylene radicals may be replaced by fluorine; SO₂-NH₂, SO₂NH(C₁-C₆)-alkyl, SO₂N[(C₁-C₆)-alkyl]₂, S-(C₁-C₆)-alkyl, S-(CH₂)ₙ-pheny), SO-(C₁-C₆)-alkyl, SO-(CH₂),-phenyl, SO₂₋(C₁-C₆)-alkyl, SO₂-(CH₂)ₙ-phenyl, where n = 0-6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(Cₗ-C₇)-acyl, phenyl, 0-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
L 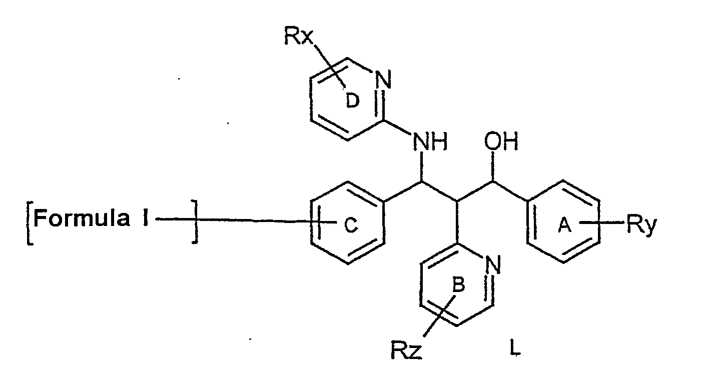
Rx, Ry, Rz independently of one another are H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where in the alkyl radicals one, more or all hydrogen(s) may be replaced by fluorine;
SO₂-NH₂, SO₂NH(C₁-C₆)-aikyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0-6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0-6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
where one of the radicals R1 or R3 must have the meaning (C₀-C₃₀)-alkylene-L, where one or more carbon atoms of the alkylene radical may be replaced by -O-, -(C=O)- or -NH-, and its pharmaceutically acceptable salts.

4. A compound of the formula I as claimed in one or more of claims 1 to 3, wherein
R1, R2, R3, R4, R5, R6 independently of one another are -(CH₂)₀₋₁₋NH-(C=O)₀₋₁-(C₃-C₂₅)-alkylene-(C=O)₀₋₁-NH-L, where one or more carbon atoms of the alkylene radical may be replaced by oxygen atoms;
H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one or more hydrogens in the alkylene radicals may be replaced by fluorine; SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂₋(C₁-C₆)-alkyl , SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂; 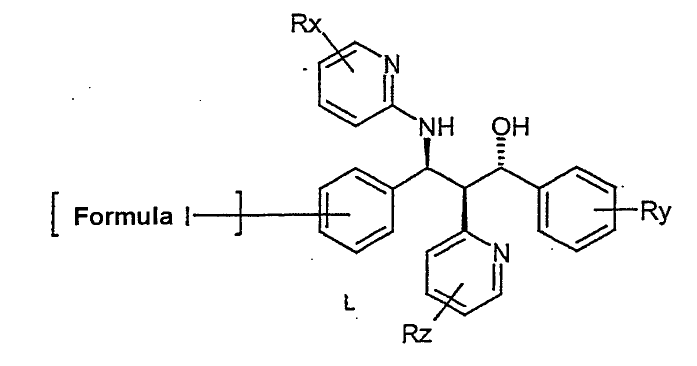
Rx, Ry, Rz independently of one another are H, F, CI, Br, I, CF₃, N0₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where in the alkyl radicals one, more or all hydrogen(s) may be replaced by fluorine;
SO₂-NH₂, SO₂NH(C₁-C₆)-alkyl , SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)n-phenyl, where n = 0-6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0-6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
where one of the radicals R1 to R3 has the meaning -(CH₂)₀₋₁-NH-(C=O)₀₋₁₋(C₃-C₂₅)-alkylene-(C=O)₀₋₁-NH-L, where one or more carbon atoms of the alkylene radical may be replaced by oxygen atoms, and its pharmaceutically acceptable salts.

5. A medicament comprising one or more of the compounds as claimed in one or more of claims 1 to 4.

6. A medicament comprising one or more compounds as claimed in one or more of claims 1 to 4 and at least one further active compound.

7. The medicament as claimed in claim 6, comprising, as further active compound, one or more compounds which normalize lipid metabolism.

8. The medicament as claimed in claim 6 or 7, which comprises, as further active compound, one or more
antidiabetics, hypoglycemically active compounds, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyases inhibitors, squalene synthetase inhibitors, lipoprotein(a) antagonists, lipase inhibitors, insulins, sulfonylureas, biguanides, meglitinides, thiolidindiones, α-glucosidase inhibitors, active compounds which act on the ATP-dependent potassium channel of the beta cells, CART agonists, NPY agonists, MC4 agonists, orexin agonists, H3 agonists, TNF agonists, CRF agonists, CRF BP agonists, urocortin agonists, β3 agonists, MSH (melanocyte-stimulating hormone) agonists, CCK agonists, serotoninreuptake inhibitors, mixed serotonin and noradrenergic compounds, 5HT agonists, bombesin agonists, galanin agonists, growth hormones, growth hormone-releasing compounds, TRH agonists, decoupling protein 2- or 3-modulators, leptin agonists, DA agonists (bromocriptine, doprexin), lipase/amylase inhibitors, PPAR modulators, RXR modulators or TR-β-agonists or amphetamines.

9. A compound as claimed in one or more of claims 1 to 4 for use as a medicament for the treatment of impaired lipid metabolism.

10. A process for preparing a medicament comprising one or more of the compounds as claimed in one or more of claims 1 to 4, which comprises mixing the active compound with a pharmaceutically acceptable carrier and bringing this mixture into a form suitable for administration.

11. The use of the compounds as claimed in one or more of claims 1 to 4 for preparing a medicament for treating hyperlipidemia.

12. The use of the compounds as claimed in one or more of claims 1 to 4 for preparing a medicament for lowering the serum cholesterol concentration.

13. The use of the compounds as claimed in one or more of claims 1 to 4 for preparing a medicament for treating arteriosclerotic symptoms.

14. The use of the compounds as claimed in one or more of claims 1 to 4 for preparing a medicament for treating insulin resistance.

## Revendications

1. Composés de formule I 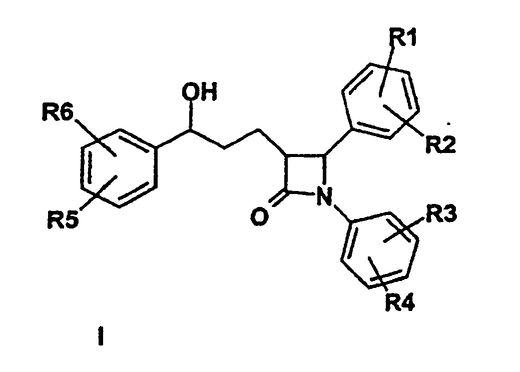 dans laquelle
R1, R2, R3, R4, R5, R6 représentent, indépendamment les uns des autres, un groupe alkylène (Cₒ-C₃ₒ) -L, un ou plusieurs atomes de carbone du radical alkylène pouvant être remplacés par -O-, (C=O) -, -CH=CH-, -C≡C-, -N [alkyle (C₁-C₆)- ou -NH- ;
H, F, CI, Br, I, CF₃, N0₂, CN, COOH, COO-alkyle (C₁-C₆), CONH₂, CONH-alkyle (Cₗ-C₆) , CON [alkyle (C₁-C₆) ] ₂, alkyle en C₁-C₆, alcényle en C₂-C₆, alcynyle en C₂-C₆, 0-alkyle(C₁-C₆), dans les radicaux alkyle un, plusieurs ou tous les atome(s) d'hydrogène pouvant être remplacé(s) par le fluor ;
SO₂-NH₂, SO₂NH-alkyle (C₁-C₆), SO₂N [alkyle (C₁-C₆)]₂, S-alkyle (C₁-C₆), S-(CH₂)ₙ-phényle, SO-alkyle (C₁-C₆), SO-(CH₂)ₙ-phényle, SO₂-alkyle (C₁-C₆), SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle(C₁-C₆), alkyle en C₁-C₆, NH₂ ;
NH₂, NH-alkyle (C₁-C₆), N- [alkyle (C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle (C₁- C₆) , alkyle en C₁-C₆, NH₂, NH-alkyle (C₁-C₆) , N [alkyle (C₁-C₆)]₂, SO₂CH₃, COOH, COO-alkyle(C₁-C₆), CONH₂ ;
L représente 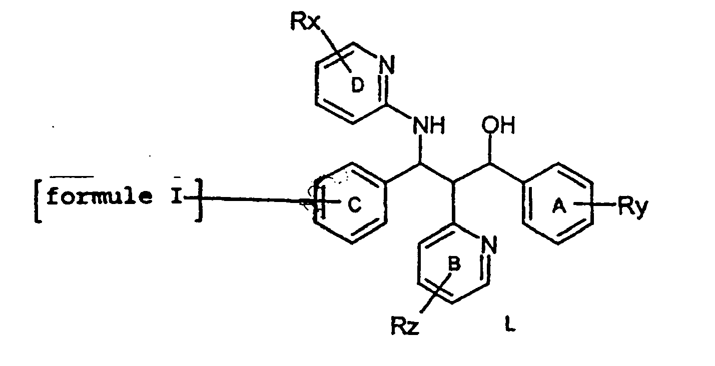
Rx, Ry, Rz représentent, chacun indépendamment, H, F, Cl, Br, I, CF₃, NO₂, CN, COOH, COO-alkyle(C₁-C₆), CONH₂, CONH-alkyle (C₁-C₆), CON[alkyle(C₁-C₆)]₂, alkyle en C₁-C₆, alcényle en C₂-C₆, alcynyle en C₂-C₆, O-alkyle(C₁-C₆), dans les radicaux alkyle un, plusieurs ou tous les atome(s) d'hydrogène pouvant être remplacé(s) par le fluor ;
SO₂-NH₂, SO₂NH-alkyle(C₁-C₆) , SO₂N[alkyle (C₁-C₆)]₂, S-alkyle(C₁-C₆), S-(CH₂)ₙ-phényle, SO-alkyle (C₁-C₆), SO-(CH₂)ₙ-phényle, SO₂-alkyle (C₁-C₆), SO₂ -(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle(C₁-C₆), alkyle en C₁-C₆, NH₂ ;
NH₂, NH-alkyle (C₁-C₆) , N- [alkyle (C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle (C₁-C₆), alkyle en C₁-C₆, NH₂, NH-alkyle (C₁-C₆), N[alkyle (C₁-C₆)]₂, SO₂CH₃, COOH, COO-alkyle(C₁-C₆), CONH₂ ;
au moins un des radicaux R1 à R6 devant toujours avoir la signification d'un groupe alkylène (C₀-C₃₀) -L, un ou plusieurs atomes de carbone du radical alkylène pouvant être remplacés par -O-, -(C=O)-, -CH=CH-, -C≡C-, -N[alkyle(C₁-C₆)]- ou -NH-,
ainsi que leurs sels pharmaceutiquement acceptables.

2. Composés de formule I, selon la revendication 1, **caractérisés en ce que** dans cette formule
R1, R2, R3, R4, R5, R6 représentent, indépendamment les uns des autres, un groupe alkylène(C₀-C₃₀)-L, un ou plusieurs atomes de carbone du radical alkylène pouvant être remplacés par -O-, -(C=O)- ou -NH- ;
H, F, Cl, Br, I, CF₃, NO₂, CN, COOH, COO-alkyle (C₁-C₆), CONH₂, CONH-alkyle (C₁-C₆), CON[alkyle(C₁-C₆)]₂, alkyle en C₁-C₆, alcényle en C₂-C₆, alcynyle en C₂-C₆, O-alkyle(C₁-C₆), dans les radicaux alkyle un, plusieurs ou tous les atome(s) d'hydrogène pouvant être remplacé(s) par le fluor ;
SO₂-NH₂, SO₂NH-alkyle (C₁-C₆), SO₂N [alkyle (C₁-C₆)]2, S-alkyle (C₁-C₆) S-(CH₂)ₙ-phényle, SO-alkyle (C₁-C₆), SO-(CH₂)ₙ-phényle, SO₂-alkyle (C₁-C₆), SO₂ - (CH₂) ₙ-phényle, n pouvant aller de 0 à 6 et le radical phényle pouvant être jusqu'à deux fois substitué par F, Cl, Br, OH, CF₃, NO₂, CN, OCF₃, O-alkyle (C₁-C₆) , alkyle en C₁-C₆, NH₂ ;
NH₂, NH-alkyle (C₁-C₆) , N- [alkyle (C₁-C₆)]₂, NH-acyle(C₁-C₇), phényle, O- (CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle (C₁-C₆), alkyle en C₁-C₆, NH₂, NH-alkyle (C₁-C₆) N [alkyle (C₁-C₆)]₂, SO₂CH₃, COOH, COO-alkyle(C₁-C₆), CONH₂ ;
L représente 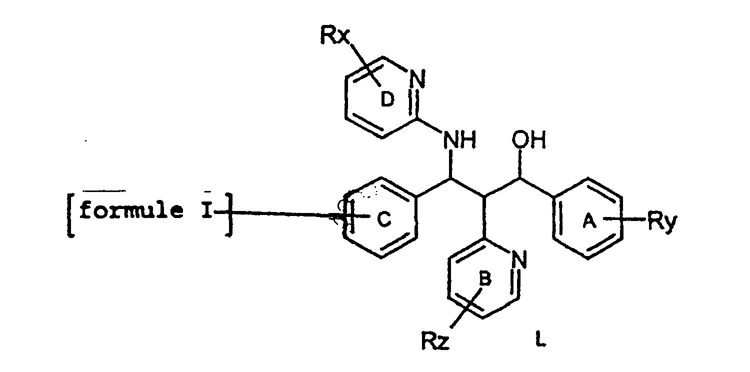
Rx, Ry, Rz représentent, chacun indépendamment, H, F, Cl, Br, I, CF₃, NO₂, CN, COOH, COO-alkyle (C₁-C₆) , CONH₂, CONH-alkyle (C₁-C₆) , CON [alkyle (C₁-C₆)]₂, alkyle en C₁-C₆, alcényle en C₂-C₆, alcynyle en C₂-C₆, O-alkyle(C₁-C₆), dans les radicaux alkyle un, plusieurs ou tous les atome(s) d'hydrogène pouvant être remplacé(s) par le fluor ;
SO₂-NH₂, SO₂NH-alkyle (C₁-C₆), SO₂N [alkyle (C₁-C₆)]₂, S-alkyle(C₁-C₆), S-(CH₂)ₙ-phényle, SO-alkyle (C₁-C₆), SO-(CH₂)ₙ-phényle , SO₂-alkyle (C₁-C₆) SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle (Cₗ-C₆), alkyle en C₁-C₆, NH₂ ;
NH₂, NH-alkyle (C₁-C₆), N- [alkyle (C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle (C₁-C₆), alkyle en C₁-C₆, NH₂, NH-alkyle(C₁-C₆), N[alkyle(C₁-C₆)]₂, SO₂CH₃, COOH, COO-alkyle(C₁-C₆), CONH₂ ;
au moins un des radicaux R1 à R6 devant toujours avoir la signification d'un groupe alkylène (Cₒ-C₃ₒ) -L, un ou plusieurs atomes de carbone du radical alkylène pouvant être remplacés par -O-, -(C=O)- ou -NH-, ainsi que leurs sels pharmaceutiquement acceptables.

3. Composés de formule I, selon la revendication 1 ou 2, **caractérisés en ce que** dans cette formule
R1, R2, R3, R4, R5, R6 représentent, indépendamment les uns des autres, un groupe alkylène (C₀-C₃₀) -L, un ou plusieurs atomes de carbone du radical alkylène pouvant être remplacés par -O-, -(C=O)- ou -NH- ;
H, F, Cl, Br, I, CF₃, NO₂, CN, COOH, COO-alkyle (C₁-C₆), CONH₂, CONH-alkyle (C₁-C₆), CON (alkyle (C₁-C₆)]₂, alkyle en C₁-C₆, alcényle en C₂-C₆, alcynyle en C₂-C₆, O-alkyle (C₁-C₆), dans les radicaux alkyle un, plusieurs ou tous les atome(s) d'hydrogène pouvant être remplacé(s) par le fluor ;
SO₂-NH₂, SO₂NH-alkyle (C₁-C₆), SO₂N [alkyle (C₁-C₆)]₂, S-alkyle(C₁-C₆), S-(CH₂)ₙ-phényle, SO-alkyle (C₁-C₆), SO-(CH₂)ₙ-phényle, SO₂-alkyle(C₁-C₆), SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle(C₁-C₆) , alkyle en C₁-C₆, NH₂ ;
NH₂, NH-alkyle (C₁-C₆), N- [alkyle (C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle(C₁-C₆), alkyle en C₁-C₆, NH₂, NH-alkyle (C₁-C₆), N [alkyle (C₁-C₆)]₂, SO₂CH₃, COOH, COO-alkyle(C₁-C₆), CONH₂;
L représente 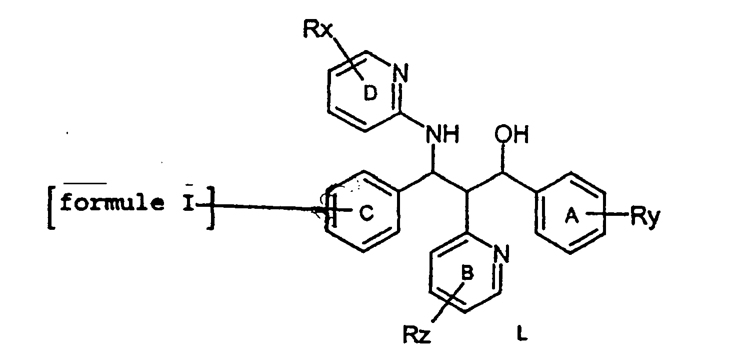
Rx, Ry, Rz représentent, chacun indépendamment, H, F, Cl, Br, I, CF₃, NO₂, CN, COOH, COO-alkyle(C₁-C₆), CONH₂, CONH-alkyle (C₁-C₆), CON[alkyle(C₁-C₆)]₂, alkyle en C₁-C₆, alcényle en C₂-C₆, alcynyle en C₂-C₆, O-alkyle (C₁-C₆), dans les radicaux alkyle un, plusieurs ou tous les atome(s) d'hydrogène pouvant être remplacé(s) par le fluor ;
SO₂-NH₂, SO₂NH-alkyle (C₁-C₆), SO₂N [alkyle (C₁-C₆)]₂, S-alkyle(C₁-C₆), S-(CH₂)ₙ-phényle, SO-alkyle (C₁-C₆), SO-(CH₂)ₙ-phényle , SO₂-alkyle(C₁-C₆), SO₂-(CH₂)ₙ-phényle, n pouvant aller de 0 à 6 et le radical phényle pouvant être jusqu'à deux fois substitué par F, Cl, Br, OH, CF₃, N0₂, CN, OCF₃, O-alkyle (C₁-C₆), alkyle en C₁-C₆, NH₂ ;
NH₂, NH-alkyle (Ci-Ce), N- [alkyle (C₁-C₆)]₂, NH-acyle (Cᵢ-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle (C₁-C₆), alkyle en C₁-C₆, NH₂, NH-alkyle (C₁-C₆), N [alkyle(C₁-C₆)]₂, SO₂CH₃, COOH, COO-alkyle(C₁-C₆), CONH₂ ;
l'un des radicaux R1 et R3 ayant la signification d'un groupe alkylène(C₀-C₃₀)-L, un ou plusieurs atomes de carbone du radical alkylène pouvant être remplacés par -O-, -(C=O)- ou -NH-, ainsi que leurs sels pharmaceutiquement acceptables.

4. Composés de formule I, selon une ou plusieurs des revendications 1 à 3, **caractérisés en ce que** dans cette formule
R1, R2, R3, R4, R5, R6 représentent, indépendamment les uns des autres, un groupement -(CH₂)₀₋₁-NH- (C=O) ₀₋₁-alkylène (C₃-C₂₅) - (C=O) ₀₋₁-NH-L, un ou plusieurs atomes de carbone du radical alkylène pouvant être remplacés par des atomes d'oxygène ;
H, F, Cl, Br, I, CF₃, NO₂, CN, COOH, COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle(C₁-C₆), dans les radicaux alkyle un, plusieurs ou tous les atome(s) d'hydrogène pouvant être remplacé(s) par le fluor ;
SO₂-NH₂, SO₂NH-alkyle (C₁-C₆), SO₂N [alkyle (C₁-C₆)]₂, S-alkyle (C₁-C₆), S-(CH₂)ₙ-phényle, SO-alkyle (C₁-C₆), SO-(CH₂)ₙ-phényle, SO₂-alkyle (C₁-C₆) SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle(C₁-C₆), alkyle en C₁-C₆, NH₂ ;
NH₂, NH-alkyle (C₁-C₆), N-[alkyle(C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle (C₁-C₆), alkyle en C₁-C₆, NH₂, NH-alkyle (C₁-C₆), N [alkyle (C₁-C₆)]₂, SO₂CH₃, COOH, COO-alkyle(C₁-C₆), CONH₂ ;
L représente 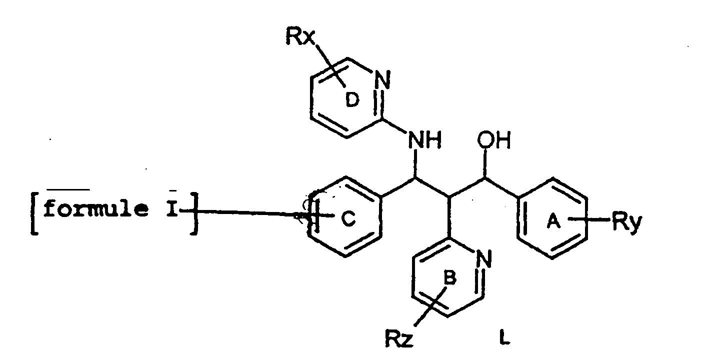
Rx, Ry, Rz représentent, chacun indépendamment, H, F, Cl, Br, I, CF₃, NO₂, CN, COOH, COO-alkyle(C₁-C₆), CONH₂, CONH-alkyle(C₁-C₆), CON [alkyle (C₁-C₆)]₂, alkyle en C₁-C₆, alcényle en C₂-C₆, alcynyle en C₂-C₆, O-alkyle (C₁-C₆), dans les radicaux alkyle un, plusieurs ou tous les atome(s) d'hydrogène pouvant être remplacé(s) par le fluor ;
SO₂-NH₂, SO₂NH-alkyle (C₁-C₆), SO₂N [alkyle (C₁-C₆)]2, S-alkyle (C₁-C₆), S-(CH₂)ₙ-phényle, SO-alkyle (C₁-C₆), SO-(CH₂)ₙ-phényle, SO₂-alkyle(C₁-C₆), SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle (C₁-C₆), alkyle en C₁-C₆, NH₂ ;
NH₂, NH-alkyle (C₁-C₆), N-[alkyle (C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle(C₁-C₆), alkyle en C₁₋C₆, NH₂, NH-alkyle (C₁-C₆), N[alkyle(C₁-C₆)]2, SO₂CH₃, COOH, COO-alkyle(C₁-C₆), CONH₂ ;
l'un des radicaux R1 et R3 ayant la signification d'un groupement
- (CH₂) ₀₋₁-NH- (C=O) ₀₋₁-alkylène (C₃-C₂₅) - (C=O) ₀₋₁-NH-L, un ou plusieurs atomes de carbone du radical alkylène pouvant être remplacés par des atomes d'oxygène;
ainsi que leurs sels pharmaceutiquement acceptables.

5. Médicament contenant un ou plusieurs des composés selon une ou plusieurs des revendications 1 à 4.

6. Médicament contenant un ou plusieurs des composés selon une ou plusieurs des revendications 1 à 4 et au moins une autre substance active.

7. Médicament selon la revendication 6, **caractérisé en ce qu'**il contient comme autre substance active un ou plusieurs composés qui régularisent le métabolisme des lipides.

8. Médicament selon la revendication 6 ou 7,
**caractérisé en ce qu'**il contient comme autre substance active un(e) ou plusieurs
antidiabétiques, principes actifs hypoglycémiants, inhibiteurs de HMGCoA-réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de résorption d'acides biliaires, inhibiteurs de CETP, adsorbeurs polymères d'acides biliaires, inducteurs de récepteurs de LDL, inhibiteurs d'ACAT, antioxydants, inhibiteurs de lipoprotéine lipase, inhibiteurs d'ATP-citrate lyase, inhibiteurs de squalène synthétase, antagonistes de lipoprotéine (a), inhibiteurs de lipase, insulines, sulfonylurées, biguanides, méglitinides, thiazolidinediones, inhibiteurs d'α-glucosidase, principes actifs agissant sur le canal potassique ATP-dépendant des cellules bêta, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, agonistes de MSH (hormone simulant les mélanocytes), agonistes de CCK, inhibiteurs de la recapture de sérotonine, composés sérotoninergiques et noradrénergiques mixtes, agonistes de 5HT, agonistes de bombésine, antagonistes de galanine, hormones de croissance, composés libérant l'hormone de croissance, agonistes de TRH, modulateurs de protéine 2 ou 3 découpleurs, agonistes de leptine, agonistes de DA (bromocriptine, doprexine), inhibiteurs de lipase/amylase, modulateurs de PPAR, modulateurs de RXR ou agonistes de TR-β ou amphétamines.

9. Composés selon une ou plusieurs des revendications 1 à 4, pour utilisation en tant que médicament destiné au traitement de troubles du métabolisme des lipides.

10. Procédé pour la fabrication d'un médicament contenant un ou plusieurs des composés selon une ou plusieurs des revendications 1 à 4, **caractérisé en ce qu'**on mélange la substance active avec un véhicule pharmaceutiquement convenable et on met ce mélange sous une forme appropriée à l'administration.

11. Utilisation des composés selon une ou plusieurs des revendications 1 à 4, pour la fabrication d'un médicament destiné au traitement de l'hyperlipidémie.

12. Utilisation des composés selon une ou plusieurs des revendications 1 à 4, pour la fabrication d'un médicament destiné à abaisser le taux de cholestérol sérique.

13. Utilisation des composés selon une ou plusieurs des revendications 1 à 4, pour la fabrication d'un médicament destiné au traitement de manifestations artérioscléreuses.

14. Utilisation des composés selon une ou plusieurs des revendications 1 à 4, pour la fabrication d'un médicament destiné au traitement de la résistance à l'insuline.
